# FASCICULE DE BREVET EUROPEEN

(11) **EP 0 566 455 B1**
(45) Date de publication et mention de la délivrance du brevet: **06.03.1996**
(21) Numéro de dépôt: 93400922.6
(22) Date de dépôt: 08.04.1993
(51) Int. Cl.: C07C 69/734

(54) **Nouveaux dérivés de l'acide 7-éthynyl alpha-(méthoxyméthylène) 1-naphtalène acétique, leur procédé de préparation et leur application comme pesticides**
Derivate der 7-Äthynyl-Alpha-(Methoxymethylen)1-naphthylessigsäure, ihr Herstellungsverfahren und ihre Anwendung als Pesticide
Derivatives of 7-ethynyle alpha-(methoxymethylene)1-naphthalenacetic acid, their process of preparation and their use as pesticides

(30) Priorité: 14.04.1992 FR 9204565
(43) Date de publication de la demande: 20.10.1993
(73) Titulaire: ROUSSEL UCLAF, F-93230 Romainville (FR)
(72) Inventeur: Benoit, Marc, F-13360 Roquevaire (FR); Brayer, Jean-Louis, F-60440 Nanteuil le Haudouin (FR); Laugraud, Sylvain, F-92410 Ville d'Avray (FR)
(74) Mandataire: Vieillefosse, Jean-Claude

(56) Documents cités:
- EP-A- 0 178 826
- EP-A- 0 267 734

## Description

La présente invention concerne de nouveaux dérivés de l'acide 7-éthynyl α-(méthoxyméthylène) 1-naphtalène acétique, leur procédé de préparation et leur application comme pesticides.

On connaissait des dérivés de l'acide α-(méthoxyméthylène) 1-naphtalène acétique doués de propriétés pesticides (cf EP-A-267734).

L'invention a pour objet les composés de formule (I) : 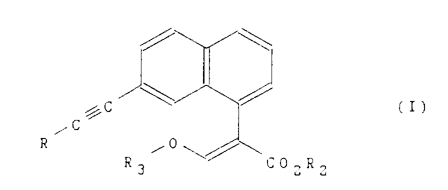 dans laquelle R représente ;
- un atome d'hydrogène ou d'halogène,
- un radical alkyle renfermant jusqu'à 8 atomes de carbone linéaire, ramifié ou cyclique saturé ou insaturé, éventuellement substitué par un ou plusieurs radicaux hydroxyle, par un ou plusieurs atomes d'halogène, éventuellement interrompu par un ou plusieurs hétéroatomes choisis parmi les atomes d'oxygène, d'azote ou de soufre,
- un radical 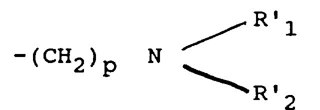 dans lequel p représente un nombre entier égal à 0, 1, 2, 3, 45, 5, 6, et R'1 et R'2 identiques ou différents représentent un atome d'hydrogène, un radical alkyle linéaire ou ramifié, renfermant jusqu'à 4 atomes de carbone,
- un radical aryle ou aryloxy renfermant jusqu'à 18 atomes de carbone éventuellement substitué sur le noyau aryle par un ou plusieurs substituants choisis parmi les atomes d'halogéne, le radical NO2, le radical C=N, les radicaux alkyle, O-alkyle et S-alkyle, linéaires, ramifiés ou cycliques, saturés ou insaturés, renfermant jusqu'à 8 atomes de carbone, éventuellement substitués par un ou plusieurs atomes d'halogène, les radicaux aryle ou aryloxy renfermant jusqu'à 16 atomes de carbone, éventuellement substitués par un ou plusieurs atomes d'halogène et les radicaux 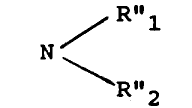 dans lesquels R''₁ et R''₂, identiques ou différents l'un de l'autre, représentent un atome d'hydrogène, un radical alkyle renfermant jusqu'à 4 atomes de carbone ou un radical aryle renfermant jusqu'à 18 atomes de carbone, éventuellement substitué par un ou plusieurs atomes d'halogène,
- un radical aryle ou aryloxy renfermant jusqu'à 18 atomes de carbone éventuellement substitué sur deux carbone adjacents du noyau aryle par un groupement méthylène ou éthylènedioxy éventuellement substitué par un ou deux atomes d'halogène,
- un radical aryle hétérocyclique à 5 ou 6 chaînons renfermant 1, 2 ou 3 hétéroatomes choisis parmi les atomes d'oxygène, de soufre et d'azote, éventuellement substitué par un ou plusieurs radicaux choisis dans le groupe constitué par les radicaux hydroxyle, NO₂, C≡N, les atomes d'halogène, les radicaux alkyle, O-alkyle et S-alkyle, renfermant jusqu'à 8 atomes de carbone, linéaires, ramifiés ou cycliques éventuellement substitués par un ou plusieurs atomes d'halogène, par un ou plusieurs radicaux hydroxyle et les radicaux 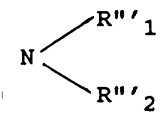 dans lesquels R'''₁ et R'''₂ identiques ou différents, représentent un atome d'hydrogène ou un radical alkyle renfermant jusqu'à 4 atomes de carbone,
- un radical 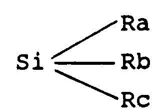 dans lequel Ra, Rb et Rc, identiques ou différents, représentent un radical alkyle linéaire, ramifié ou cyclique renfermant jusqu'à 4 atomes de carbone, éventuellement substitué par un ou plusieurs atomes d'halogène, un radical aryle éventuellement substitué par un ou plusieurs atomes d'halogène, un ou plusieurs radicaux hydroxyle, un ou plusieurs radicaux alkyle, linéaires ou ramifiés, éventuellement substitués par un ou plusieurs atomes d'halogène, un ou plusieurs radicaux O-alkyle ou S-alkyle, éventuellement substitués par un ou plusieurs atomes d'halogène,
   ou R représente un radical 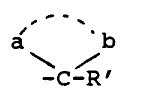 dans lequel a et b, identiques ou différents, représentent un atome d'hydrogène, un atome d'halogène, un radical hydroxyle, un radical alkyle linéaire ou ramifié renfermant jusqu'à 6 atomes de carbone éventuellement substitué par un ou plusieurs atomes d'halogène ou un radical alkoxy linéaire ou ramifié renfermant jusqu'à 6 atomes de carbone, ou a et b forment ensemble avec l'atome de carbone auquel ils sont liés un radical cycloalkyle renfermant jusqu'à 6 atomes de carbone,
   et R' représente l'un des valeurs indiquées ci-dessus pour R, R₂ et R₃ identiques ou différents l'un de l'autre représentent un radical alkyle renfermant jusqu'à 8 atomes de carbone, linéaire, ramifié ou cyclique, éventuellement substitué par un ou plusieurs atomes d'halogène, ou par un radical 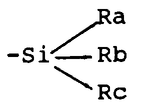 dans lequel Ra, Rb et Rc ont les significations indiquées précédemment.

Dans la définition des différents substituants :
- alkyle représente de préférence, un radical méthyle, éthyle, propyle, isopropyle, butyle, isobutyle, sec-butyle, tert-butyle,
- cycloalkyle représente de préférence cyclopropyle, cyclobutyle, cyclopentyle,
- alkyle insaturé représente de préférence éthényle, éthynyle, propényle, propynyle, butényle, butynyle.

Lorsque le radical alkyle linéaire ou ramifié est interrompu par un ou plusieurs hétéroatomes, il s'agit de préférence d'un ou plusieurs atomes d'oxygène ou d'azote.

Lorsqu'il s'agit d'un radical cycloalkyle interrompu par un plusieurs hétéroatomes, il s'agit de préférence d'un hétérocycle azoté lié par un atome d'azote tel que pyrrolidine, pyrazoline, pipéridine, pipérazine ou morpholine,
- aryle représente de préférence le radical phényle,
- aryle hétérocyclique représente de préférence un radical thiazolyle, oxazolyle, isothiazolyle, isoxazolyle, thiadiazolyle, oxadiazolyle (1,2,4) ou (1,3,4), triazolyle, imidazolyle, pyridynyle, pyridinyle ou pyrazolyle,
- si R ou R' représente un halogène, il s'agit de préférence d'un atome de brome ou de chlore,
- si R ou R' représente un radical lui-même substitué par un atome d'halogène, l'halogène est de préférence un atome de fluor, de chlore ou de brome.

L'invention a plus particulièrement pour objet, les composés de formule (I) dans lesquels le radical R₂ représente un radical méthyle ainsi que ceux dans lesquels R₃ représente un radical méthyle.

La géométrie de la double liaison exo (éther d'énol) est E ou Z et l'invention qui concerne les produits E et les produits Z, ainsi que les mélanges E+Z, a plus spécialement pour objet les composés dans lesquels la géométrie de la double liaison exo (éther d'énol) est E.

Parmi les composés préférés de l'invention, on peut citer
- les composés de formule (I) dans lesquels R représente un radical alkyle renfermant jusqu'à 6 atomes de carbone,
- les composés de formule (I) dans lesquels R représente un radical terbutyle, ainsi que ceux dans lesquels R représente un radical butyle,
- les composés de formule (I) dans lesquels R représente un radical 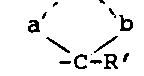 dans lequel a et b identiques ou différents représentent un atome d'hydrogène, un radical hydroxyle, un radical alkyle linéaire ou ramifié renfermant jusqu'à 4 atomes de carbone ou a et b forment ensemble avec l'atome de carbone auquel ils sont liés un radical cycloalkyle renfermant jusqu'à 4 atomes de carbone et R' a la signification indiquée ci-dessus et notamment ceux dans lesquels a et b identiques, représentent un atome d'hydrogène, un radical méthyle ou forment ensemble avec l'atome de carbone auquel ils sont liés, un radical cyclopropyle,
- les composés de formule (I) dans lesquels R ou R' représente un radical 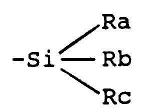 dans lequel Ra, Rb et Rc identiques ou différents représentent un radical alkyle renfermant jusqu'à 4 atomes de carbone et notamment ceux dans lesquels Ra, Rb et Rc représentent un radical méthyle,
- les composés de formule (I) dans lesquels R ou R' représente un radical phényle éventuellement substitué par un ou plusieurs radicaux choisis dans le groupe constitué par les atomes d'halogène, les radicaux hydroxyle, les radicaux alkyle ou alkoxy renfermant jusqu'à 4 atomes de carbone, les radicaux méthylène et éthylènedioxy et le radical CF₃ notamment ceux dans lesquels le noyau phényle est non substitué ou substitué par un ou plusieurs atomes de chlore ou de brome, par un ou plusieurs radicaux CF₃, éthoxy ou par un radical méthylènedioxy.

Parmi les composés préférés de l'invention, on peut citer les composés dont la préparation est donnée ci-après dans la partie expérimentale et tout spécialement les composés des exemples 1 à 4, 15, 16, 17, 29, 39, 40, 51, 52 et 53.

Les composés de formule (I) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent également être utilisés pour lutter contre les insectes dans les locaux, pour lutter notamment contre les mouches, les moustiques et les blattes.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 5 g et 300 g de matière active à l'hectare.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques du genre de Boophilus, ceux du genre Hyalomnia, ceux du genre Amblyomnia et ceux du genre Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment le produit de l'exemple 1 à 4, 15, 16, 17, 29, 39, 40, 51, 52 et 53.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de matière active ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2.2.1]hept-5-ène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet un procédé de préparation des composés de formule (I), caractérisé en ce que l'on soumet un composé de formule (II) : 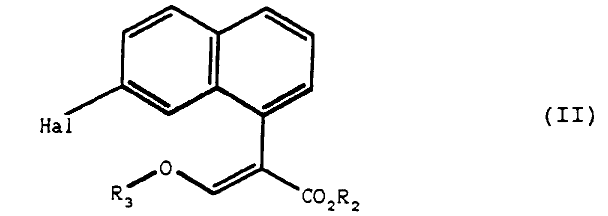 dans laquelle Hal représente un atome d'halogène et R₂ et R₃ conservent la même signification que précédemment à l'action d'un composé de formule (III) :

RC≡CH (III)

dans laquelle R conserve sa signification précédente, pour obtenir le composé de formule (I), que l'on soumet, si désiré et si R comporte une fonction hydroxyle, à un agent de fonctionnalisation pour obtenir le composé de formule (I) correspondant.

Dans un mode de réalisation préféré du procédé de l'invention, la réaction entre le composé de formule (II) et le composé de formule (III) est réalisée au sein d'un solvant dipolaire aprotique comme l'acétonitrile, en présence d'une amine tertiaire comme la triéthylamine, de palladium métallique supporté par du charbon, d'une phosphine tertiaire comme la triphénylphosphine et d'un catalyseur à base de cuivre comme l'iodure cuivreux.

L'invention a également pour objet un procédé de préparation des composés de formule (I) telle que définie ci-dessus caractérisé en ce que l'on soumet en outre un composé de formule (I) dans laquelle R représente un atome d'hydrogène à l'action d'un composé de formule ArHal, dans laquelle Ar représente un radical aryle ou hétéroaryle éventuellement substitué et Hal représente un atome d'halogène pour obtenir le composé de formule (I) correspondant dans laquelle R représente un radical aryle ou hétéroaryle éventuellement substitué.

Les composés de formule (II) sont des produits nouveaux et sont en eux-mêmes un objet de la présente invention.

Les composés de formule (II) peuvent être préparés à partir des composés de formule (IV) : 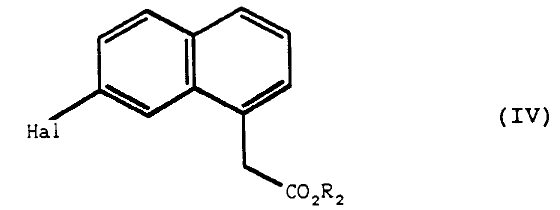 par action d'une base forte et d'un formiate d'alkyle.

La préparation 1 décrit un exemple détaillé de préparation de produit de formule (II) et l'invention a tout spécialement pour objet ce composé de formule (II) de la préparation.

Les produits de formule (IV) sont décrits et revendiqués dans la demande de brevet français 91 12516 déposée le 11 octobre 1991, par la société demanderesse, un exemple de préparation de formule (IV) est donné ci-après dans la partie expérimentale.

La préparation des composés de formule (IV) peut être schématisée comme suit :

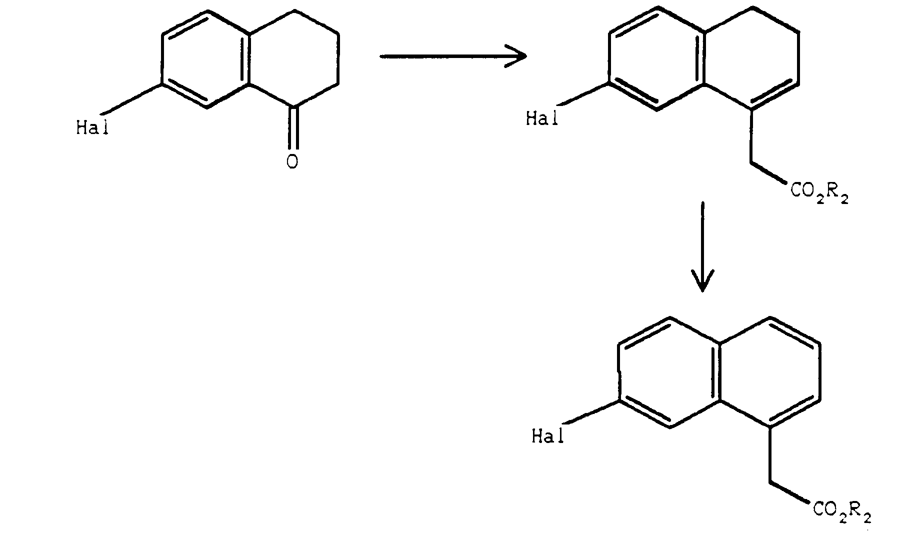

Les composés de formule (I) dans lesquels R est un groupement 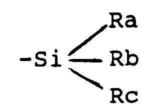 sont des produits biologiquement actifs et peuvent également servir de produits intermédiaires pour préparer d'autres produits biologiquement actifs selon les schémas réactionnels suivants :

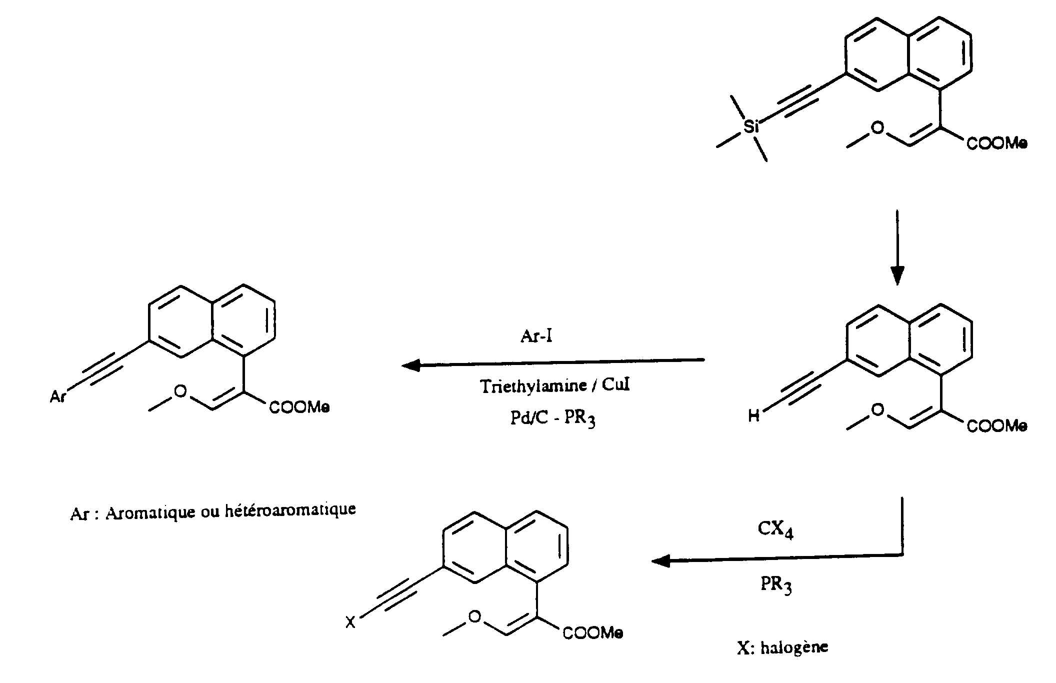

Les composés de formule (I), dans lesquels le radical R comporte une fonction hydroxyle, peuvent être fonctionnalisés à l'aide d'un réactif alkylant tel l'iodure de méthyle ou le sulfate diméthylique pour conduire aux dérivés méthoxy correspondants. Ils peuvent aussi être soumis à l'action du D.A.S.T. (diméthyl amino sulfure trifluorure) pour conduire aux dérivés dans lesquels R comporte au moins un atome de fluor.

Les exemples suivants illustrent l'invention sans toutefois la limiter.

### EXEMPLE 1: (E) 7-(3,3-diméthyl 1-butynyl) α-(méthoxyméthylène) 1-naphtalène acétate de méthyle

On introduit 1,5 g du produit de la préparation 1, dans un mélange anhydre de 15 cm³ de triéthylamine et 10 cm³ d'acétonitrile. On ajoute 0,198 g de palladium à 10 % sur charbon actif, 0,035 g d'iodure de cuivre, 0,195 g de triphénylphosphine et 0,60 cm³ de terbutylacétylène. On porte la suspension obtenue au reflux pendant 5 heures, on ajoute 1 cm³ de terbutylacétylène. On porte à nouveau au reflux pendant 2 heures. On filtre et amène à sec. On obtient 2,62 g d'un produit que l'on chromatographie sur silice en éluant avec le mélange hexane-chlorure de méthylène (50-50). On isole la fraction de rf = 0,20, concentre au rotavapor, rince, sèche à 50°C. On obtient 0,99 g d'un produit que l'on recristallise dans l'acétate d'éthyle. On obtient ainsi 0,630 g de produit recherché. F = 172°C.
δ (CDCl₃) = 7,79 ppm.

En opérant comme précédemment, on a obtenu les produits suivants à partir du même produit de formule (II) et du produit RC≡CH correspondant.
* Les points de fusion sont exprimés en degrés Celsius et sont déterminés à l'aide d'un appareil METTLER PF62.
** Les déplacements chimiques (δ) sont exprimés en ppm à partir d'une référence interne le tétraméthylsilane et déterminés à l'aide d'un appareil de Résonance Magnétique Nucléaire BRUCKER à 250 MHz.

### EXEMPLE 2: (E) α-(méthoxyméthylène) 7-[2-(triméthylsilyl) éthynyl] 1-naphtalène acétate de méthyle

F = 157°C. δ (CDCl₃) = 7,79 ppm.

### EXEMPLE 3: (E) 7-(1-hexynyl) α-(méthoxyméthylène) 1-naphtalène acétate de méthyle

F = 88°C. δ (CDCl₃) = 7,78 ppm.

### EXEMPLE 4: (E) 7-[2-[1-(4-éthoxyphényl) cyclopropyl] éthynyl] α-(méthoxyméthylène) 1-naphtalène acétate de méthyle

F = 145°C. δ (CDCl₃) = 7,78 ppm.

### EXEMPLE 5: (E) α-(méthoxyméthylène) 7-(phényléthynyl) 1-naphtalène acétate de méthyle

F = 161°C. δ (CDCl₃) = 7,81 ppm.

### EXEMPLE 6: (E) 7-(3-hydroxy 3-méthyl 1-butynyl) α-(méthoxyméthylène) 1-naphtalène acétate de méthyle

F = 117°C. δ (CDCl₃) = 7,80 ppm.

### EXEMPLE 7: (E) 7-(3-méthoxy 3-méthyl 1-butynyl) α-(méthoxyméthylène) 1-naphtalène acétate de méthyle

F = 134°C. δ (CDCl₃) = 7,80 ppm.

### EXEMPLE 8: (E) 7-(3-hydroxy 3-phényl 1-propynyl) α-(méthoxyméthylène) 1-naphtalène acétate de méthyle

F = 192°C. δ (CDCl₃) = 7,78 ppm.

### EXEMPLE 9: (E) 7-(3-hydroxy 3-phényl 1-butynyl) α-(méthoxyméthylène) 1-naphtalène acétate de méthyle

F = 169°C. δ (CDCl₃) = 7,79 ppm.

### EXEMPLE 10: (E) 7-(3-méthyl 1-butynyl) α-(méthoxyméthylène) 1-naphtalène acétate de méthyle

F = 114°C. δ (CDCl₃) = 7,78 ppm.

### EXEMPLE 11: (E) α-(méthoxyméthylène) 7-(cyclopentyl éthynyl) 1-naphtalène acétate de méthyle

F = 80°C. δ (CDCl₃) = 7,78 ppm.

### EXEMPLE 12: (E) 7-(3-fluoro 3-méthyl 1-butynyl) α-(méthoxyméthylène) 1-naphtalène acétate de méthyle

En faisant réagir le produit obtenu à l'exemple 6 avec du DAST (diméthyl amino sulfure trifluorure) à -10°C dans le tétrahydrofuranne, on a obtenu le produit recherché.

F = 74°C. δ (CDCl₃) = 7,80 ppm.

### EXEMPLE 13: (E) α-(méthoxyméthylène) 7-(éthynyl) 1-naphtalène acétate de méthyle

On ajoute à -50°C, 18,1 cm³ d'une solution de BU₄NF 1N dans le THF, dans une solution renfermant 6,15 g de (E) α-(méthoxyméthylène) 7-[2-(triméthylsilyl) éthynyl] 1-naphtalène acétate de méthyle (préparé à l'exemple 2) et 180 cm³ de THF. On maintient le mélange réactionnel sous agitation pendant 2 heures. On le verse sur une solution aqueuse de dihydrogénophosphate de potassium. On extrait au chlorure de méthylène. On sèche, évapore le solvant et obtient 6,1 g d'un produit que l'on chromatographie sur silice en éluant avec le mélange hexane-acétate d'éthyle (9-1). On obtient 4,2 g de produit recherché. F = 140°C. δ (CDCl₃) = 7,78 ppm.

### EXEMPLE 14: (E) α-(méthoxyméthylène) 7-(bromoéthynyl) 1-naphtalène acétate de méthyle

On ajoute, à 20°C, 3,5 g de triphénylphosphine, dans une solution renfermant 0,6 g du produit de l'exemple 13, 2,24 g de tétrabromure de carbone et 6 cm³ de chlorure de méthylène. On maintient le mélange réactionnel sous agitation pendant 15 minutes. On verse dans l'eau, extrait au chlorure de méthylène, sèche et évapore. On obtient 6,2 g d'un produit que l'on reprend dans un mélange renfermant 20 cm³ de chlorure de méthylène et 10 cm³ d'éther éthylique. On filtre le précipité obtenu et concentre le filtrat. On obtient 2,1 g d'un produit que l'on chromatographie sur silice en éluant avec le mélange hexane-acétate d'éthyle (8-2). On obtient ainsi 0,31 g de produit recherché, sous forme d'huile. δ (CDCl₃) = 7,78 ppm.

### EXEMPLE 15: (E) 7-[2-(4-chlorophényl) éthynyl] α-(méthoxyméthylène) naphtalène acétate de méthyle

A une solution de 1 g du produit de l'exemple 13 et de 1 g de bromo 4-chlorobenzène dans 10 cm³ de triéthylamine et 6 cm³ d'acétonitrile anhydres sont ajoutés 0,15 g de palladium à 10% sur charbon actif, 0,13 g de triphénylphosphine et 0,03 g d'iodure cuivreux. La suspension résultante est chauffée à reflux sous atmoshère d'azote pendant 2 heures 30 minutes puis filtrée. Le filtrat est dilué par du chlorure de méthylène et lavé par une solution aqueuse d'acide chlorhydrique 2N, séché sur MgSO₄ puis évaporé sous pression réduite. Le résidu est chromatographié sur silice (éluant : CH₂Cl₂-hexane 7-3) pour donner 0,75 g de produit recherché. F = 162°C.

### EXEMPLE 16: (E) 7-[2-(4-bromophényl) éthynyl] α-(méthoxyméthylène) naphtalène acétate de méthyle

Une solution de 20 cm³ de diméthylformamide contenant 1,5 g de produit de l'exemple 13, 1,6 g de iodo 4-bromobenzène, 0,025 g de dichloro bis(triphénylphosphine) palladium, 0,023 g d'iodure cuivreux, 1,1 cm³ de triéthylamine est agitée à 20°C, sous atmosphère d'azote, pendant 6 heures puis versée dans de l'eau et extraite par de l'éther diisopropylique. La phase organique est séchée sur MgSO₄ et évaporée sous pression réduite. Le résidu est chromatographié sur gel de silice (éluant : CH₂Cl₂-hexane 8-2) pour donner 2,03 g de produit recherché. F = 173°C.

En opérant comme précédemment, on a préparé les produits suivants :

### EXEMPLE 17 : (E) α-(méthoxyméthylène) 7-[3-méthyl 3-phényl 1-butynyl] naphtalène acétate de méthyle

F = 136°C.

### EXEMPLE 18 : (E) 7-[2-(3-chlorophényl) éthynyl] α-(méthoxyméthylène) naphtalène acétate de méthyle

F = 194°C.

### EXEMPLE 19 : (E) α-(méthoxyméthylène) 7-[2-(4-pyridinyl) éthynyl] naphtalène acétate de méthyle

F = 194°C.

### EXEMPLE 20 : (E) α-(méthoxyméthylène) 7-[2-(4-phénoxyphényl) éthynyl] naphtalène acétate de méthyle

F = 182°C.

### EXEMPLE 21 : (E) 7-[3-[(2-méthoxy) éthoxy] méthoxy 1-propynyl] α-(méthoxyméthylène) naphtalène acétate de méthyle

F = 76°C.

### EXEMPLE 22 : (E) 7-[2-(2-chlorophényl) éthynyl] α-(méthoxyméthylène) naphtalène acétate de méthyle

F = 146°C.

### EXEMPLE 23 : (E) α-(méthoxyméthylène) 7-[3-méthyl 3-(1-pipéridinyl) 1-butynyl] naphtalène acétate de méthyle

F = 145°C.

### EXEMPLE 24 : (E) α-(méthoxyméthylène) 7-[2-(3-pyridinyl) éthynyl] naphtalène acétate de méthyle

F = 142°C.

### EXEMPLE 25 : (E) α-(méthoxyméthylène) 7-[2-(2-pyridinyl) éthynyl] naphtalène acétate de méthyle

F = 148°C.

### EXEMPLE 26 : (E) α-(méthoxyméthylène) 7-[3-(4-méthylphénoxy) 3-méthyl 1-butynyl] naphtalène acétate de méthyle

F < 50°C.

### EXEMPLE 27 : (E) α-(méthoxyméthylène) 7-[3-méthyl 3-(méthylpropylamino) 1-butynyl] naphtalène acétate de méthyle

F = 128°C.

### EXEMPLE 28 : (E) 7-[3-(4-chlorophénoxy) 3-méthyl 1-butynyl] α-(méthoxyméthylène) naphtalène acétate de méthyle

F = 114°C.

### EXEMPLE 29 : (E) 7-[2-(3-bromophényl) éthynyl] α-(méthoxyméthylène) naphtalène acétate de méthyle

F = 204°C.

### EXEMPLE 30 : (E) α-(méthoxyméthylène) 7-[3-méthyl 3-(méthylamino) 1-butynyl] naphtalène acétate de méthyle

F = 152°C.

### EXEMPLE 31 : (E) 7-[2-[3-(4-fluorophényl) phényl] éthynyl] α-(méthoxyméthylène) naphtalène acétate de méthyle

F = 164°C.

### EXEMPLE 32 : (E) 7-[3,3-diméthyl 1-butynyl] α-[[1-(méthyl) éthoxy] méthylène] naphtalène acétate de méthyle

F = 111°C.

### EXEMPLE 33 : (E) α-(éthoxyméthylène) 7-[3,3-diméthyl 1-butynyl] naphtalène acétate de méthyle

F = 98°C.

### EXEMPLE 34 : (E) α-(méthoxyméthylène) 7-[3-méthyl 3-(phénylamino) 1-butynyl] naphtalène acétate de méthyle

F = 154°C.

### EXEMPLE 35 : (E) 7-[3-[[(2-méthoxy) éthoxy] méthoxy] 3-méthyl 1-butynyl] α-(méthoxyméthylène) naphtalène acétate de méthyle

F = 67°C.

### EXEMPLE 36 : (E) 7-[3,3-diméthyl 1-butynyl] α-(méthoxyméthylène) naphtalène acétate de 2-triméthylsilyl) éthyle

F = 105°C.

### EXEMPLE 37 : (E) 7-[2-(4-fluorophényl) éthynyl] α-(méthoxyméthylène) naphtalène acétate de méthyle

F = 170°C.

### EXEMPLE 38 : (E) 7-[2-(4-méthoxyphényl) éthynyl] α-(méthoxyméthylène) naphtalène acétate de méthyle

F = 164°C.

### EXEMPLE 39 : (E) α-(méthoxyméthylène) 7-[3-(triméthylsilyl) 1-propynyl] naphtalène acétate de méthyle

F = 107°C.

### EXEMPLE 40 : (E) 7-[3-(4-chlorophényl) 3-méthyl 1-butynyl] α-(méthoxyméthylène) naphtalène acétate de méthyle

F = 152°C.

### EXEMPLE 41 : (E) 7-[2-(3,4-dichlorophényl) éthynyl] α-(méthoxyméthylène) naphtalène acétate de méthyle

F = 200°C.

### EXEMPLE 42 : (E) 7-[3-(4-chlorophényl) amino] 3-méthyl 1-butynyl] α-(méthoxyméthylène) naphtalène acétate de méthyle

F = 149° C.

### EXEMPLE 43 : (E) α-(méthoxyméthylène) 7-[3-[méthyl (phénylamino) 3-méthyl] 1-butynyl] naphtalène acétate de méthyle

F = 158°C.

### EXEMPLE 44 : (E) 7-[2-[4-(1,1-diméthyléthyl) phényl] éthynyl] α-(méthoxyméthylène) naphtalène acétate de méthyle

F = 182°C.

### EXEMPLE 45 : (E) α-(méthoxyméthylène) 7-[2-(4-méthylphényl) éthynyl] naphtalène acétate de méthyle

F = 164°C.

### EXEMPLE 46 : (E) 7-[2-(4-brome 2-fluorophényl) éthynyl] α-(méthoxyméthylène) naphtalène acétate de méthyle

F = 135°C.

### EXEMPLE 47 : (E) 7-[2-(3-chlore 4-fluorophényl) éthynyl] α-(méthoxyméthylène) naphtalène acétate de méthyle

F = 210°C.

### EXEMPLE 48 : (E) 7-[2-(3,5-dichlorophényl) éthynyl] α-méthoxyméthylène) naphtalène acétate de méthyle

F = 180°C.

### EXEMPLE 49 : (E) α-(méthoxyméthylène) 7-[2-[4-(trifluorométhoxy) phényl] éthynyl] naphtalène acétate de méthyle

F = 150°C.

### EXEMPLE 50 : (E) 7-(3,3-diméthylbutynyl] α-méthoxyméthylène) naphtalène acétate de méthyle

F = 138°C.

### EXEMPLE 51 : (E) α-(méthoxyméthylène) 7-[2-[4-(trifluoro

### méthyl) phényl] éthynyl] naphtalène acétate de méthyle

F = 157°C.

### EXEMPLE 52 : (E) 7-[2-(3,5-bis(trifluorométhyl) phényl] éthynyl] α-(méthoxyméthylène) naphtalène acétate de méthyle

F = 192°C.

### EXEMPLE 53 : (E) 7-[2-(1,3-benzodioxol-5-yl) éthynyl] α-(méthoxyméthylène) naphtalène acétate de méthyle

F = 181°C.

### EXEMPLE 54 : (E) 7-[3-(diméthylamino) 3-méthyl 1-butynyl) α-(méthoxyméthylène) naphtalène acétate de méthyle

F = 148°C.

### EXEMPLE 55 : (E) 7-(1-propynyl) α-(méthoxyméthylène) naphtalène acétate de méthyle

F = 109°C.

### EXEMPLE 56 : (E) 7-[3-[(4-chlorophényl) méthylamino] 3-méthyl 1-butynyl] α-(méthoxyméthylène) naphtalène acétate de méthyle

F = 179°C.

### EXEMPLE 57 : (E) 7-[2-(4-cyanophényl) éthynyl] α-(méthoxyméthylène) naphtalène acétate de méthyle

F = 129°C.

### EXEMPLE 58 : (E) 7-[2-(2,4-dichlorophényl) éthynyl] α-(méthoxyméthylène) naphtalène acétate de méthyle

F = 156°C.

### EXEMPLE 59 : (E) 7-[2-(2,3-dichlorophényl) éthynyl] α-(méthoxyméthylène) naphtalène acétate de méthyle

F = 153°C.

### EXEMPLE 60 : (E) 7-[2-(2,5-dichlorophényl) éthynyl] α-(méthoxyméthylène) naphtalène acétate de méthyle

F = 162°C.

### EXEMPLE 61 : (E) 7-[3-(1,1-diméthyléthoxy) 3-méthyl 1-butynyl] α-(méthoxyméthylène) naphtalène acétate de méthyle

F = 140°C.

### PREPARATION 1 : 1-(E) 7-bromo (α-méthoxyméthylène) 1-naphtalène acétate de méthyle

On ajoute une solution renfermant 5,99 g de produit de la préparation 2, 70 cm³ de DMF et 28,5 cm³ de formiate de méthyle dans une solution renfermant 2,02 g d'hydrure de sodium et 20 cm³ de DMF anhydre. On maintient le mélange réactionnel sous agitation pendant 35 minutes. On verse sur une solution aqueuse d'acide chlorhydrique, extrait à l'éther, sèche, filtre et concentre. On obtient 9,54 g d'un produit que l'on reprend dans 60 cm³ d'acétone. On ajoute 8,70 g de carbonate de potassium et 2,4 cm³ de sulfate de diméthyle. On maintient le mélange réactionnel sous agitation à 20°C pendant 16 heures. On verse dans l'eau. On essore et rince le produit obtenu. On obtient 8,67 g d'un produit que l'on chromatographie sur silice en éluant avec le mélange hexane-chlorure de méthylène-acétone (90-5-5). On obtient ainsi 5,08 g de produit fondant à 149°C.

### PREPARATION 2 : 7-bromo 1-naphtalène acétate de méthyle

### STADE A : 7-bromo 3,4-dihydro 1-naphtalène acétate de méthyle

On chauffe à 60°C une suspension renfermant 5,8 g de poudre de zinc activé (c'est-à-dire obtenue après lavages à l'acide chlorhydrique à 20 % (en volume) puis à 5 % puis à l'eau, puis à l'éthanol et enfin à l'éther éthylique et séchage), 70 cm³ de tétrahydrofuranne anhydre, 1 cristal d'iode et 0,1 cm³ de bromoacétate de méthyle. On ajoute sous atmosphère d'azote, une solution renfermant 10 g de 7-bromotétralone (préparé selon J. Org. Chem., 27, 76 (1962)) 5 cm³ de bromoacétate de méthyle et 60 cm³ de tétrahydrofuranne anhydre. On agite ensuite le milieu réactionnel à 20°C pendant 2 heures, verse dans l'eau, neutralise par adjonction de chlorure d'ammonium et extrait à l'éther. On filtre la solution aqueuse et réextrait à l'éther. On rassemble les phases organiques, les lave avec une solution aqueuse saturée en chlorure de sodium, sèche et évapore sous pression réduite. On obtient 13,6 g d'un produit que l'on dissout dans 90 cm³ d'acide trifluoroacétique. On chauffe à 50°C pendant 30 minutes et verse dans une solution refermant 1 l d'eau et 500 cm³ d'éther. On ajoute du carbonate de potassium jusqu'à un pH voisin de 10. On décante, lave avec une solution aqueuse saturée en chlorure de sodium, sèche et évapore sous pression réduite. On obtient 11,8 g de produit que l'on chromatographie sur silice en éluant avec un mélange hexane-éther isopropylique (9-1) puis avec un mélange hexane-éther isopropylique (7-3). On obtient 9,9 g du produit recherché.
CCM (silice) éluant : hexane-éther isopropylique (8-2)
rf = 0,38.
Spectre de RMN (250 MHz)

| | |
|---|---|
| CH₂ en 3 | ∼ 2,32 (m) |
| CH₂ en 4 | 2,73 (t) |
| =C-CH₂-C=O | 3,41 (s) |
| CO₂CH₃ | 3,71 (s) |
| H₂ | 6,04 (t) |
| H₅ | 7,00 (d) |
| H₈ + H₆ | ∼ 7,25 (m) |

### STADE B : 7-bromo 1-naphtalène acétate de méthyle

On chauffe à 90°C pendant 3 h 30 minutes une solution renfermant 9,85 g de produit préparé au stade A avec 10,3 g de dichlorodicyanobenzoquinone et 300 cm³ de toluène. On maintient le mélange réactionnel pendant 12 heures à 20°C. On filtre, et évapore sous pression réduite pour obtenir 12,7 g de produit que l'on chromatographie sur silice en éluant avec le mélange hexane-chlorure de méthylène (7-3). On obtient ainsi 5,1 g de produit recherché. F = 61°C.
Spectre de RMN (250 MHz)

| | |
|---|---|
| COOCH₃ | 3,66 (s) |
| =C₆H₃-CH₂₋CO | 3,98 (s) |
| H₈ | 8,1 (sl) |
| | 7,42 (m) 2H |
| H aromatiques | 7,52 (dd) 1H |
| | 7,68 (m) 2H |

### Etude de l'activité des composés de l'invention

**a) Etude de l'activité sur Aphis Craccivora**
   Des plants de fèves sont traités par trempage des feuilles dans une solution hydroacétonique de matière active (50 % acétone, 50 % eau) puis séchées sous hotte aspirante.
   Les feuilles sont ensuite infestées : 20 femelles d'Aphis Craccivora adultes par feuille et maintenues à 22°C sous plafond lumineux.
   Les contrôles de mortalité sont effectués au bout de 48 heures.
**b) Etude de l'activité acaricide sur Tetranychus Urticae**
   On utilise des plants de haricot comportant 2 feuilles infestées de 30 femelles de Tetranychus Urticae par feuille et mis sous bonette aérée sous plafond lumineux en lumière constante. Les plants sont traités au pistolet Fisher : 4 ml de solution toxique par plant d'un mélange à volume égal d'eau et d'acétone. On laisse sécher pendant une 1/2 heure puis on procède à l'infestation. Les contrôles de mortalité sont effectués au bout de 3 jours.
c) Etude de l'effet sur larves de Spodoptera Littoralis par contact et ingestion.
   On utilise des larves du stade L3 de Spodoptera Littoralis. On opère à 22°C dans des conditions d'humidité relative de 50 %. On utilise des boîtes de PETRI renfermant un rond de papier filtre humidifié, dans chaque boîte on place deux feuilles de haricot traitées par une solution hydroacétonique (50-50) renfermant le produit à tester.
   On fait le comptage des larves mortes au bout de 7 jours.
d) Etude de l'effet sur Phaedon Cochleariae. On opère à 22°C dans des conditions d'humidité relative de 50 %. On utilise des boîtes de PETRI renfermant un rond de papier filtre humidifié et deux disques de feuille de chou chinois traités par une solution hydroacétonique (50-50) renfermant le produit à tester.
   On fait le comptage des insectes morts au bout d'une semaine.
**Résultats** : Dès la dose de 100 ppm, les produits de l'invention présentent une activité insecticide intéressante.

## Revendications

1. Les composés de formule (I) : 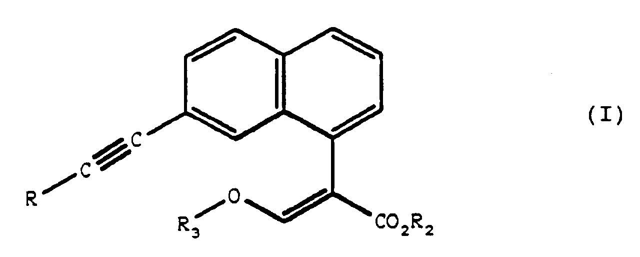 dans laquelle R représente :
- un atome d'hydrogène ou d'halogène,
- un radical alkyle renfermant jusqu'à 8 atomes de carbone linéaire, ramifié ou cyclique saturé ou insaturé, éventuellement substitué par un ou plusieurs radicaux hydroxyle, par un ou plusieurs atomes d'halogène, éventuellement interrompu par un ou plusieurs hétéroatomes choisis parmi les atomes d'oxygène, d'azote ou de soufre,
- un radical 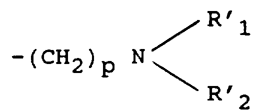 dans lequel p représente un nombre entier égal à 0, 1, 2, 3, 4, 5 ou 6, et R'₁ et R'₂ identiques ou différents représentent un atome d'hydrogène, un radical alkyle linéaire ou ramifié, renfermant jusqu'à 4 atomes de carbone,
- un radical aryle ou aryloxy renfermant jusqu'à 18 atomes de carbone éventuellement substitué sur le noyau aryle par un ou plusieurs substituants choisis parmi les atomes d'halogène, le radical NO₂, le radical C≡N, les radicaux alkyle, O-alkyle et S-alkyle, linéaires, ramifiés ou cycliques, saturés ou insaturés, renfermant jusqu'à 8 atomes de carbone, éventuellement substitués par un ou plusieurs atomes d'halogène, les radicaux aryle ou aryloxy renfermant jusqu'à 16 atomes de carbone, éventuellement substitués par un ou plusieurs atomes d'halogène et les radicaux 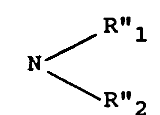 dans lesquels R"₁ et R"₂, identiques ou différents l'un de l'autre, représentent un atome d'hydrogène, un radical alkyle renfermant jusqu'à 4 atomes de carbone ou un radical aryle renfermant jusqu'à 18 atomes de carbone, éventuellement substitué par un ou plusieurs atomes d'halogène,
- un radical aryle ou aryloxy renfermant jusqu'à 18 atomes de carbone éventuellement substitué sur deux carbone adjacents du noyau aryle par un groupement méthylène ou éthylènedioxy éventuellement substitué par un ou deux atomes d'halogène,
- un radical aryle hétérocyclique à 5 ou 6 chaînons renfermant 1, 2 ou 3 hétéroatomes choisis parmi les atomes d'oxygène, de soufre et d'azote, éventuellement substitué par un ou plusieurs radicaux choisis dans le groupe constitué par les radicaux hydroxyle, NO₂, C≡N, les atomes d'halogène, les radicaux alkyle, O-alkyle et S-alkyle, renfermant jusqu'à 8 atomes de carbone, linéaires, ramifiés ou cycliques éventuellement substitués par un ou plusieurs atomes d'halogène, par un ou plusieurs radicaux hydroxyle et les radicaux 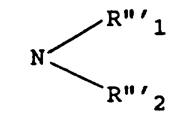 dans lesquels R"'₁ et R"'₂ identiques ou différents, représentent un atome d'hydrogène ou un radical alkyle renfermant jusqu'à 4 atomes de carbone,
- un radical 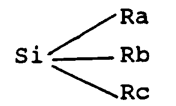 dans lequel Ra, Rb et Rc, identiques ou différents, représentent un radical alkyle linéaire, ramifié ou cyclique renfermant jusqu'à 4 atomes de carbone, éventuellement substitué par un ou plusieurs atomes d'halogène, un radical aryle éventuellement substitué par un ou plusieurs atomes d'halogène, un ou plusieurs radicaux hydroxyle, un ou plusieurs radicaux alkyle, linéaires ou ramifiés, éventuellement substitués par un ou plusieurs atomes d'halogène, un ou plusieurs radicaux O-alkyle ou S-alkyle, éventuellement substitués par un ou plusieurs atomes d'halogène,
ou R représente un radical 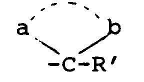 dans lequel a et b, identiques ou différents, représentent un atome d'hydrogène, un atome d'halogène, un radical hydroxyle, un radical alkyle linéaire ou ramifié renfermant jusqu'à 6 atomes de carbone éventuellement substitué par un ou plusieurs atomes d'halogène ou un radical alkoxy linéaire ou ramifié renfermant jusqu'à 6 atomes de carbone, ou a et b forment ensemble avec l'atome de carbone auquel ils sont liés un radical cycloalkyle renfermant jusqu'à 6 atomes de carbone,
et R' représente l'un des valeurs indiquées ci-dessus pour R, R₂ et R₃ identiques ou différents l'un de l'autre représentent un radical alkyle renfermant jusqu'à 8 atomes de carbone,
linéaire, ramifié ou cyclique, éventuellement substitué par un ou plusieurs atomes d'halogène, ou par un radical 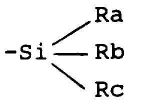 dans lequel Ra, Rb et Rc ont les significations indiquées précédemment.

2. Les composés de formule (I) tels que définis à la revendication 1 dans lesquels le radical R₂ représente un radical méthyle.

3. Les composés de formule (I) tels que définis à la revendication 1 ou 2 dans lesquels R₃ représente un radical méthyle.

4. Les composés de formule (I) tels que définis à la revendication 1 à 3 dans lesquels la géométrie de la double liaison exo (éther d'énol) est E.

5. Les composés de formule (I) tels que définis à l'une quelconque des revendications 1 à 4 dans lesquels R représente un radical alkyle renfermant jusqu'à 6 atomes de carbone.

6. Les composés de formule (I) tels que définis à la revendication 5 dans lesquels R représente un radical n-butyl ou terbutyle.

7. Les composés de formule (I) tels que définis à l'une quelconque des revendications 1 à 4 dans lesquels R représente un radical 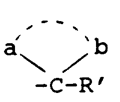 dans lequel a et b identiques ou différents représentent un atome d'hydrogène, un radical hydroxyle, un radical alkyle linéaire ou ramifié renfermant jusqu'à 4 atomes de carbone ou a et b forment ensemble avec l'atome de carbone auquel ils sont liés un radical cycloalkyle renfermant jusqu'à 4 atomes de carbone et R' a la signification indiquée à la revendication 1.

8. Les composés de formule (I) tels que définis à la revendication 7 dans lesquels a et b identiques, représentent un atome d'hydrogène, un radical méthyle ou forment ensemble avec l'atome de carbone auquel ils sont liés, un radical cyclopropyle,

9. Les composés de formule (I) tels que définis à l'une quelconque des revendications 1 à 4, 7 et 8 dans lesquels R ou R' représente un radical 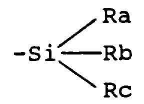 dans lequel Ra, Rb et Rc identiques ou différents représente un radical alkyle renfermant jusqu'à 4 atomes de carbone.

10. Les composés de formule (I) tels que définis à la revendication 9, dans lesquels Ra, Rb et Rc représentent un radical méthyle.

11. Les composés de formule (I) tels que définis à l'une quelconque des revendications 1 à 4, 7 et 8, dans lesquels R ou R' représente un radical phényle éventuellement substitué par un ou plusieurs radicaux choisis dans le groupe constitué par les atomes d'halogène, les radicaux hydroxyle, les radicaux alkyle ou alkoxy renfermant jusqu'à 4 atomes de carbone, les radicaux méthylène et éthylènedioxy et le radical CF₃.

12. Les composés de formule (I) tels que définis à la revendication 11 dans lesquels le noyau phényle est non substitué ou substitué par un ou plusieurs atomes de chlore ou de brome, par un ou plusieurs radicaux CF₃, éthoxy ou par un radical méthylènedioxy.

13. Les composés de formule (I) tels que définis à la revendication 1 dont les noms suivent :
- (E) 7-(3,3-diméthyl 1-butynyl) α-(méthoxyméthylène) 1-naphtalène acétate de méthyle,
- (E) α-(méthoxyméthylène) 7-[2-(triméthylsilyl) éthynyl] 1-naphtalène acétate de méthyle,
- (E) 7-(1-hexynyl) α-(méthoxyméthylène) 1-naphtalène acétate de méthyle,
- (E) 7-[2-[1-(4-éthoxy phényl) cyclopropyl] éthynyl] α-(méthoxy méthylène) 1-naphtalène acétate de méthyle,
- (E) 7-[2-(4-chlorophényl) éthynyl] α-(méthoxyméthylène) naphtalène acétate de méthyle,
- (E) 7-[2-(4-bromophényl) éthynyl] α-(méthoxyméthylène) naphtalène acétate de méthyle,
- (E) α-(méthoxyméthylène) 7-[3-méthyl 3-phényl 1-butynyl] naphtalène acétate de méthyle,
- (E) 7-[2-(3-bromophényl) éthynyl] α-(méthoxyméthylène) naphtalène acétate de méthyle,
- (E) α-(méthoxyméthylène) 7-[3-(triméthylsilyl) 1-propynyl] naphtalène acétate de méthyle,
- (E) 7-[3-(4-chlorophényl) 3-méthyl 1-butynyl] α-méthoxyméthylène) naphtalène acétate de méthyle,
- (E) α-(méthoxyméthylène) 7-[2-[4-(trifluorométhyl) phényl] éthynyl] naphtalène acétate de méthyle,
- (E) 7-[2-(3,5-bis(trifluorométhyl) phényl] éthynyl] α-(méthoxyméthylène) naphtalène acétate de méthyle,
- (E) 7-[2-(1,3-benzodioxol-5-yl) éthynyl] α-(méthoxyméthylène) naphtalène acétate de méthyle.

14. Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 12.

15. Les compositions destinées à la lutte contre les parasites des végétaux, les parasites des locaux et les parasites des animaux à sang chaud, caractérisées en ce qu'elles renferment comme principe actif au moins un des produits définis à la revendication 13.

16. Les compositions insecticides renfermant comme principe actif au moins l'un des produits définis à l'une quelconque des revendications 1 à 13.

17. Les compositions acaricides renfermant comme principe actif au moins l'un des produit définis à l'une quelconque des revendications 1 à 13.

18. Les compositions nématicides renfermant comme principe actif au moins d'un des produits définis à l'une quelconque des revendications 1 à 13.

19. Les compositions acaricides destinées à la lutte contre les parasites des animaux à sang chaud, notamment contre les tiques et les gales, caractérisées en ce qu'elles renferment comme principe actif, au moins l'un des produits définis à l'une quelconque des revendications 1 à 13.

20. Procédé de préparation des composés de formule (I) tels que définis à l'une quelconque des revendications 1 à 13, caractérisé en ce que l'on soumet un composé de formule (II) : 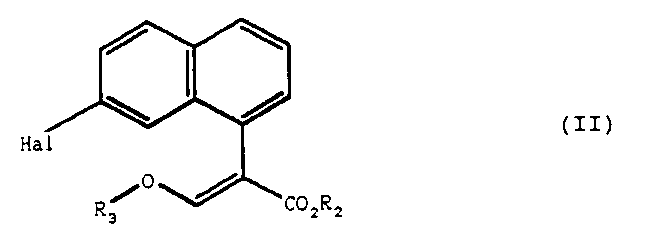 dans laquelle Hal représente un atome d'halogène et R₂ et R₃ conservent la même signification que précédemment à l'action d'un composé de formule (III) :
RC≡CH (III)
dans laquelle R conserve sa signification précédente, pour obtenir le composé de formule (I) que l'on soumet, si désiré et si R comporte une fonction hydroxyle, à un agent de fonctionnalisation pour obtenir le composé de formule (I) correspondant.

21. Procédé selon la revendication 20, caractérisé en ce que l'on soumet en outre un composé de formule (I) dans laquelle R représente un atome d'hydrogène à l'action d'un composé de formule ArHal, dans laquelle Ar représente un radical aryle ou hétéroaryle éventuellement substitué et Hal représente un atome d'halogène pour obtenir le composé de formule (I) correspondant dans laquelle R représente un radical aryle ou hétéroaryle éventuellement substitué.

22. A titre de produits chimiques nouveaux, les composés de formule (II) tels que définis à la revendication 20.

23. A titre de produit chimique nouveau tel que défini à la revendication 22, le 7-bromo α-(méthoxyméthylène) 1-naphtalène acétate de méthyle.

## Patentansprüche

1. Die Verbindungen der Formel (I) 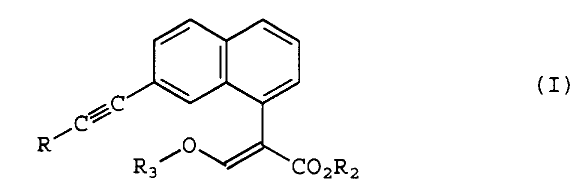 in der R darstellt:
- ein Wasserstoff- oder Halogenatom,
- einen linearen, verzweigten oder cyclischen, gesättigten oder ungesättigten Alkylrest mit bis zu 8 Kohlenstoffatomen, gegebenenfalls substituiert durch einen oder mehrere Hydroxylreste, durch ein oder mehrere Halogenatome und gegebenenfalls unterbrochen durch ein oder mehrere Heteroatome, ausgewählt unter den Atomen von Sauerstoff, Stickstoff oder Schwefel,
- einen Rest 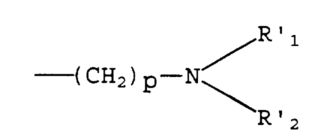 worin p eine ganze Zahl von 0, 1, 2, 3, 4, 5 oder 6 darstellt und R'₁ und R'₂, gleich oder verschieden, ein Wasserstoffatom oder einen linearen oder verzweigten Alkylrest mit bis zu 4 Kohlenstoffatomen bedeuten,
- einen Aryl- oder Aryloxyrest mit bis zu 18 Kohlenstoffatomen, gegebenenfalls substituiert an dem Arylkern durch einen oder mehrere Substituenten, ausgewählt unter den Halogenatomen, dem Rest NO₂, dem Rest C≡N, den linearen, verzweigten oder cyclischen, gesättigten oder ungesättigten Resten Alkyl, O-Alkyl und S-Alkyl mit bis zu 8 Kohlenstoffatomen, gegebenenfalls substituiert durch ein oder mehrere Halogenatome,
wobei die Aryl- oder Aryloxyreste, die bis zu 16 Kohlenstoffatome umfassen, gegebenenfalls substituiert sind durch ein oder mehrere Halogenatome und die Reste 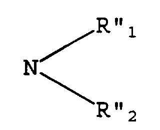 worin R"₁ und R"₂, untereinander gleich oder verschieden, ein Wasserstoffatom, einen Alkylrest mit bis zu 4 Kohlenstoffatomen oder einen Arylrest mit bis zu 18 Kohlenstoffatomen bedeuten, gegebenenfalls substituiert durch ein oder mehrere Halogenatome,
- einen Aryl- oder Aryloxyrest mit bis zu 18 Kohlenstoffatomen, gegebenenfalls substituiert an zwei angrenzenden Kohlenstoffen des Arylkerns durch eine Methylen- oder Ethylendioxygruppe, gegebenenfalls substituiert durch ein oder zwei Halogenatome,
- einen heterocyclischen Arylrest mit 5 oder 6 Ringgliedern, der 1, 2 oder 3 Heteroatome umfaßt, ausgewählt unter den Atomen von Sauerstoff, Schwefel oder Stickstoff, gegebenenfalls substituiert durch einen oder mehrere Reste, gewählt aus der durch die Reste Hydroxyl, NO₂, C≡N, die Halogenatome, die linearen, verzweigten oder cyclischen Reste Alkyl, O-Alkyl und S-Alkyl mit bis zu 8 Kohlenstoffatomen gebildeten Gruppe, gegebenenfalls substituiert durch ein oder mehrere Halogenatome, durch einen oder mehrere Hydroxylreste und die Reste 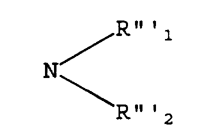 worin R"'₁ und R"'₂, gleich oder verschieden, ein Wasserstoffatom oder einen Alkylrest mit bis zu 4 Kohlenstoffatomen darstellen,
- einen Rest 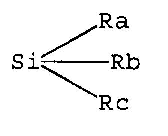 worin Ra, Rb und Rc, gleich oder verschieden, einen linearen, verzweigten oder cyclischen Alkylrest mit bis zu 4 Kohlenstoffatomen darstellen, gegebenenfalls substituiert durch ein oder mehrere Halogenatome, einem Arylrest, gegebenenfalls substituiert durch ein oder mehrere Halogenatome, einen oder mehrere Hydroxylreste, einen oder mehrere lineare oder verzweigte Alkylreste, ge- gebenenfalls substituiert durch ein oder mehrere Halogenatome, einen oder mehrere O-Alkyl- oder B-Alkylreste, gegebenenfalls substituiert durch ein oder mehrere Halogenatome,
oder R einen Rest 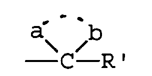 darstellt, worin a und b, gleich oder verschieden, ein Wasserstoffatom, ein Halogenatom, einen Hydroxylrest, einen linearen oder verzweigten Alkylrest mit bis zu 6 Kohlenstoffatomen, gegebenenfalls substituiert durch ein oder mehrere Halogenatome oder einen linearen oder verzweigten Alkoxyrest mit bis zu 6 Kohlenstoffatomen bedeuten, oder a und b bilden zusammen mit dem Kohlenstoffatom, an das sie gebunden sind, einen Cycloalkylrest mit bis zu 6 Kohlenstoffatomen,
und R' einen der vorstehend für R angegebenen Werte aufweist, R₂ und R₃, untereinander gleich oder verschieden, einen linearen, verzweigten oder cyclischen Alkylrest mit bis zu 8 Kohlenstoffatomen darstellen, gegebenenfalls substituiert durch ein oder mehrere Halogenatome oder durch einen Rest 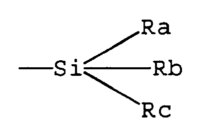 worin Ra, Rb und Rc die oben angegebenen Bedeutungen besitzen.

2. Verbindungen der Formel (I) wie in Anspruch 1 definiert, worin der Rest R₂ einen Methylrest darstellt.

3. Verbindungen der Formel (I) wie in Anspruch 1 oder 2 definiert, worin R₃ einen Methylrest darstellt.

4. Verbindungen der Formel (I) wie in Anspruch 1 bis 3 definiert, worin die Geometrie der Doppelbindung exo (Enolether) E ist.

5. Verbindungen der Formel (I) wie in irgendeinem der Ansprüche 1 bis 4 definiert, worin R einen Alkylrest mit bis zu 6 Kohlenstoffatomen darstellt.

6. Verbindungen der Formel (I) wie in Anspruch 5 definiert, worin R einen n-Butyl- oder tert.-Butylrest darstellt.

7. Verbindungen der Formel (I) wie in irgendeinem der Ansprüche 1 bis 4 definiert, worin R einen Rest 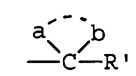 darstellt, in dem a und b, gleich oder verschieden, ein Wasserstoffatom, einen Hydroxylrest oder einen linearen oder verzweigten Alkylrest mit bis zu 4 Kohlenstoffatomen bedeuten, oder a und b zusammen mit dem Kohlenstoffatom, an das sie gebunden sind, einen Cycloalkylrest mit bis zu 4 Kohlenstoffatomen bilden und R' die in Anspruch 1 angegebene Bedeutung besitzt.

8. Verbindungen der Formel (I) wie in Anspruch 7 definiert, worin a und b gleich sind und ein Wasserstoffatom oder einen Methylrest darstellen, oder zusammen mit dem Kohlenstoffatom, an das sie gebunden sind, einen Cyclopropylrest bilden.

9. Verbindungen der Formel (I) wie in irgendeinem der Ansprüche 1 bis 4, 7 und 8 definiert, worin R oder R' einen Rest 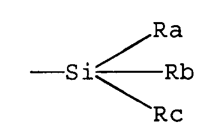 darstellen, worin Ra, Rb und Rc, gleich oder verschieden, einen Alkylrest mit bis zu 4 Kohlenstoffatomen bedeuten.

10. Verbindungen der Formel (I) wie in Anspruch 9 definiert, worin Ra, Rb und Rc einen Methylrest darstellen.

11. Verbindungen der Formel (I) wie in irgendeinem der Ansprüche 1 bis 4, 7 und 8 definiert, worin R oder R' einen Phenylrest darstellen, gegebenenfalls substituiert durch einen oder mehrere Reste, gewählt aus der durch die Halogenatome, die Hydroxylreste, die Alkyl- oder Alkoxyreste mit bis zu 4 Kohlenstoffatomen, die Reste Methylen und Ethylendioxy und den Rest CF₃ gebildeten Gruppe.

12. Verbindungen der Formel (I) wie in Anspruch 11 definiert, worin der Phenylkern nicht substituiert oder substituiert ist durch ein oder mehrere Atome von Chlor oder Brom, durch einen oder mehrere Reste CF₃, Ethoxy oder durch einen Methylendioxyrest.

13. Verbindungen der Formel (I) wie in Anspruch 1 definiert, deren Namen folgen:
- (E)-7-(3,3-Dimethyl-1-butinyl) -α-(methoxymethylen)-1-naphthalin-essigsäure-methylester,
- (E)-7-[2-(Trimethylsilyl)-ethinyl]-α-(methoxymethylen)-1-naphthalin-essigsäure-methylester,
- (E)-7-(1-Hexinyl)-α-(methoxymethylen)-1-naphthalin-essigsäuremethylester,
- (E)-7-{2-[1-(4-Ethoxyphenyl)-cyclopropyl]-ethinyl}-α-(methoxymethylen)-1-naphthalin-essigsäure-methylester,
- (E)-7-[2-(4-Chlorphenyl)-ethinyl]-α-(methoxymethylen)-naphthalin-essigsäure-methylester,
- (E)-7-[2-(4-Bromphenyl)-ethinyl]-α-(methoxymethylen)-naphthalin-essigsäure-methylester,
- (E)-7-[(3-Methyl-3-phenyl)-1-butinyl]-α-(methoxymethylen)-naphthalin-essigsäure-methylester,
- (E)-7-[2-(3-Bromphenyl)-ethinyl]-α-(methoxymethylen)-naphthalin-essigsäure-methylester,
- (E)-7-[3-(Trimethylsilyl)-1-propinyl]-α-(methoxymethylen)-naphthalin-essigsäure-methylester,
- (E)-7-[3-(4-Chlorphenyl)-3-methyl-1-butinyl]-α-(methoxymethylen)-naphthalin-essigsäure-methylester,
- (E)-7-{2-[4-(Trifluormethyl)-phenyl]-ethinyl)-α-(methoxymethylen)-naphthalin-essigsäure-methylester,
- (E)-7-{2-[3,5-Bis-(Trifluormethyl)-phenyl]-ethinyl}-α-(methoxymethylen)-naphthalin-essigsäure-methylester,
- (E)-7-[2-(1,3-Benzodioxol-5-yl)-ethinyl]-α-(methoxymethylen)-naphthalin-essigsäure-methylester.

14. Zusammensetzungen zur Bekämpfung von Parasiten an Pflanzen, Parasiten in Räumen und Parasiten an Warmblüter-Tieren, dadurch gekennzeichnet, daß sie als Wirkstoff mindestens eines der in irgendeinem der Ansprüche 1 bis 12 definierten Produkte umfassen.

15. Zusammensetzungen zur Bekämpfung von Parasiten an Pflanzen, Parasiten in Räumen und Parasiten an Warmblüter-Tieren, dadurch gekennzeichnet, daß sie als Wirkstoff mindestens eines der in Anspruch 13 definierten Produkte umfassen.

16. Insektizide Zusammensetzungen, umfassend als Wirkstoff mindestens eines der in irgendeinem der Ansprüche 1 bis 13 definierten Produkte.

17. Akarizide Zusammensetzungen, umfassend als Wirkstoff mindestens eines der in irgendeinem der Ansprüche 1 bis 13 definierten Produkte.

18. Nematizide Zusammensetzungen, umfassend als Wirkstoff mindestens eines der in irgendeinem der Ansprüche 1 bis 13 definierten Produkte.

19. Akarizide Zusammensetzungen zur Bekämpfung von Parasiten an Warmblüter-Tieren, insbesondere von Zecken und Räude, dadurch gekennzeichnet, daß sie als Wirkstoff mindestens eines der in irgendeinem der Ansprüche 1 bis 13 definierten Produkte umfassen.

20. Verfahren zur Herstellung der Verbindungen der Formel (I) wie in irgendeinem der Ansprüche 1 bis 13 definiert, dadurch gekennzeichnet, daß man eine Verbindung der Formel (II) 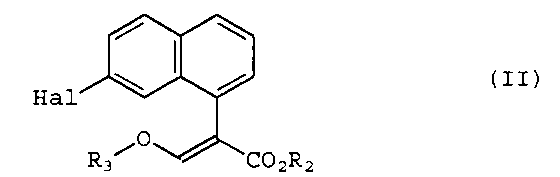 in der Hal ein Halogenatom darstellt und R₂ und R₃ die gleiche Bedeutung wie vorstehend behalten, der Einwirkung einer Verbindung der Formel (III)
RC≡CH (III)
unterzieht, in der R seine vorstehende Bedeutung behält, um die Verbindung der Formel (I) zu erhalten, die man, wenn erwünscht und wenn R eine Hydroxylfunktion umfaßt, einem Funktionalisierungs-Mittel unterzieht, um die entsprechende Verbindung der Formel (I) zu erhalten.

21. Verfahren nach Anspruch 20, dadurch gekennzeichnet, daß man außerdem eine Verbindung der Formel (I), in der R ein Wasserstoffatom darstellt, der Einwirkung einer Verbindung der Formel ArHal unterzieht, in der Ar einen gegebenenfalls substituierten Arylrest oder Heteroarylrest darstellt und Hal ein Halogenatom bedeutet, um die entsprechende Verbindung der Formel (I) zu erhalten, in der R einen gegebenenfalls substituierten Arylrest oder Heteroarylrest darstellt.

22. Als chemisch neue Produkte die Verbindungen der Formel (II), wie in Anspruch 20 definiert.

23. Als chemisch neues Produkt wie in Anspruch 22 definiert, der 7-Brom-α-(methoxymethylen)-1-naphthalin-essigsäure-methylester.

## Claims

1. The compounds of formula (I): 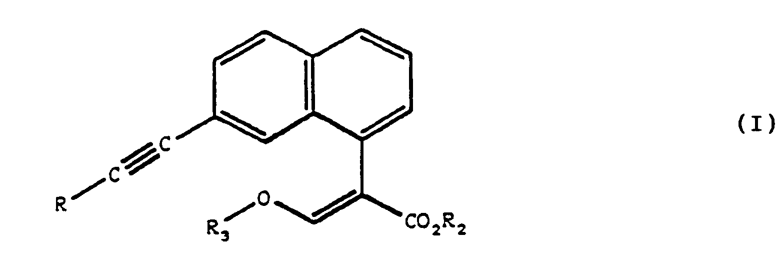 in which R represents:
- a hydrogen or halogen atom,
- a saturated or unsaturated linear, branched or cyclic alkyl radical containing up to 8 carbon atoms, optionally substituted by one or more hydroxyl radicals, by one or more halogen atoms, optionally interrupted by one or more heteroatoms chosen from oxygen, nitrogen or sulphur atoms,
- a 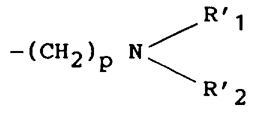 in which p represents an integer equal to 0, 1, 2, 3, 4, 5 or 6, and R'₁ and R'₂ identical or different represent a hydrogen atom, a linear or branched alkyl radical containing up to 4 carbon atoms,
- an aryl or aryloxy radical containing up to 18 carbon atoms optionally substituted on the aryl nucleus by one or more substituents chosen from halogen atoms, the NO₂ radical, the C≡N radical, saturated or unsaturated, linear, branched or cyclic alkyl, O-alkyl and S-alkyl radicals containing up to 8 carbon atoms, optionally substituted by one or more halogen atoms, aryl or aryloxy radicals containing up to 16 carbon atoms, optionally substituted by one or more halogen atoms and 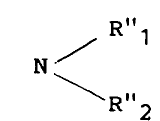 radicals
in which R"₁ and R"₂, identical to or different from each other, represent a hydrogen atom, an alkyl radical containing up to 4 carbon atoms or an aryl radical containing up to 18 carbon atoms, optionally substituted by one or more halogen atoms,
- an aryl or aryloxy radical containing up to 18 carbon atoms optionally substituted on two adjacent carbons of the aryl nucleus by a methylene or ethylenedioxy group optionally substituted by one or two halogen atoms,
- a heterocyclic aryl radical with 5 or 6 links containing 1, 2 or 3 heteroatoms chosen from oxygen, sulphur and nitrogen atoms, optionally substituted by one or more radicals chosen from the group constituted by hydroxyl, NO₂, C≡N radicals, halogen atoms, linear, branched or cyclic alkyl, O-alkyl and S-alkyl radicals containing up to 8 carbon atoms, optionally substituted by one or more halogen atoms, by one or more hydroxyl radicals and 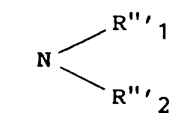 radicals
in which R"'₁ and R"'₂, identical or different, represent a hydrogen atom or an alkyl radical containing up to 4 carbon atoms,
- an 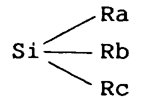 radical
in which Ra, Rb and Rc, identical or different, represent a linear, branched or cyclic alkyl radical containing up to 4 carbon atoms, optionally substituted by one or more halogen atoms, an aryl radical optionally substituted by one or more halogen atoms, one or more hydroxyl radicals, one or more linear or branched alkyl radicals, optionally substituted by one or more halogen atoms, one or more O-alkyl or S-alkyl radicals, optionally substituted by one or more halogen atoms,
or R represents a 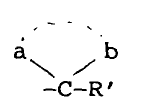 radical in which a and b, identical or different, represent a hydrogen atom, a halogen atom, a hydroxyl radical, a linear or branched alkyl radical containing up to 6 carbon atoms optionally substituted by one or more halogen atoms or a linear or branched alkoxy radical containing up to 6 carbon atoms, or a and b form together with the carbon atom to which they are linked a cycloalkyl radical containing up to 6 carbon atoms,
and R' represents one of the values indicated above for R, R₂ and R₃, identical to or different from each other, represent a linear, branched or cyclic alkyl radical containing up to 8 carbon atoms, optionally substituted by one or more halogen atoms or
by an 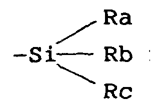 radical in which Ra, Rb and Rc have the meanings indicated previously.

2. The compounds of formula (I) as defined in claim 1 in which the R₂ radical represents a methyl radical.

3. The compounds of formula (I) as defined in claim 1 or 2 in which R₃ represents a methyl radical.

4. The compounds of formula (I) as defined in claims 1 to 3 in which the geometry of the exo (enol ether) double bond is E.

5. The compounds of formula (I) as defined in any one of claims 1 to 4 in which R represents an alkyl radical containing up to 6 carbon atoms.

6. The compounds of formula (I) as defined in claim 5 in which R represents an n-butyl or terbutyl radical.

7. The compounds of formula (I) as defined in anv one of claims 1 to 4 in which R represents a 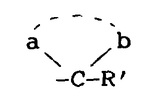 radical in which a and b, identical or different, represent a hydrogen atom, a hydroxyl radical, a linear or branched alkyl radical containing up to 4 carbon atoms or a and b form together with the carbon atom to which they are linked a cycloalkyl radical containing up to 4 carbon atoms and R ' has the meaning indicated in claim 1.

8. The compounds of formula (I) as defined in claim 7 in which a and b are identical and represent a hydrogen atom, a methyl radical or form together with the carbon atom to which they are linked, a cyclopropyl radical.

9. The compounds of formula (I) as defined in any one of claims 1 to 4, 7 and 8 in which R or R' represents an 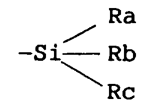 radical in which Ra, Rb and Rc, identical or different, represent an alkyl radical containing up to 4 carbon atoms.

10. The compounds of formula (I) as defined in claim 9, in which Ra, Rb and Rc represent a methyl radical.

11. The compounds of formula (I) as defined in any one of claims 1 to 4, 7 and 8, in which R or R' represents a phenyl radical optionally substituted by one or more radicals chosen from the group constituted by halogen atoms, hydroxyl radicals, alkyl or alkoxy radicals containing up to 4 carbon atoms, methylene and ethylenedioxy radicals and the CF₃ radical.

12. The compounds of formula (I) as defined in claim 11 in which the phenyl nucleus is non-substituted or substituted by one or more chlorine or bromine atoms, by one or more CF₃ radicals, ethoxy or by a methylenedioxy radical.

13. The compounds of formula (I) as defined in claim 1 whose names follow:
- methyl (E) 7-(3,3-dimethyl 1-butynyl) alpha-(methoxymethylene) 1-naphthalene acetate,
- methyl (E) alpha-(methoxymethylene) 7-[2-(trimethylsilyl) ethynyl] 1-naphthalene acetate,
- methyl (E) 7-(1-hexynyl) alpha-(methoxymethylene) 1-naphthalene acetate,
- methyl (E) 7-[2-[1-(4-ethoxy phenyl) cyclopropyl] ethynyl] alpha-methoxymethylene) 1-naphthalene acetate,
- methyl (E) 7-[2-(4-chlorophenyl) ethynyl] alpha-(methoxymethylene) naphthalene acetate,
- methyl (E) 7-[2-(4-bromophenyl) ethynyl] alpha-(methoxymethylene) naphthalene acetate,
- methyl (E) alpha-(methoxymethylene) 7-[3-methyl 3-phenyl 1-butynyl] naphthalene acetate,
- methyl (E) 7-[2-(3-bromophenyl) ethynyl] alpha-(methoxymethylene) naphthalene acetate,
- methyl (E) alpha-(methoxymethylene) 7-[3-(trimethylsilyl) 1-propynyl] naphthalene acetate,
- methyl (E) 7-[3-(4-chlorophenyl) 3-methyl 1-butynyl] alphamethoxy- methylene) naphthalene acetate,
- methyl (E) alpha-(methoxymethylene) 7-[2-[4-(trifluoromethyl) phenyl] ethynyl] naphthalene acetate,
- methyl (E) 7-[2-(3,5-bis(trifluoromethyl) phenyl] ethynyl] alpha-(methoxymethylene) naphthalene acetate,
- methyl (E) 7-[2-(1,3-benzodioxol-5-yl) ethynyl] alpha(methoxy -methylene) naphthalene acetate.

14. The compositions intended to combat parasites of vegetation, parasites of premises and parasites of warm-blooded animals, characterized in that they contain at least one of the products defined in any one of claims 1 to 12 as active ingredient.

15. The compositions intended to combat parasites of vegetation, parasites of premises and parasites of warm-blooded animals, characterized in that they contain at least one of the products defined in claim 13 as active ingredient.

16. The insecticide compositions containing as active ingredient at least one of the products defined in any one of claims 1 to 13.

17. The acaricide compositions containing as active ingredient at least one of the products defined in any one of claims 1 to 13.

18. The nematicide compositions containing as active ingredient at least one of the products defined in any one of claims 1 to 13.

19. The acaricide compositions intended to combat parasites of warm-blooded animals, notably ticks and mites, characterized in that they contain as active ingredient, at least one of the products defined in any one of claims 1 to 13.

20. Preparation process for the compounds of formula (I) as defined in any one of claims 1 to 13, characterized in that a compound of formula (II): 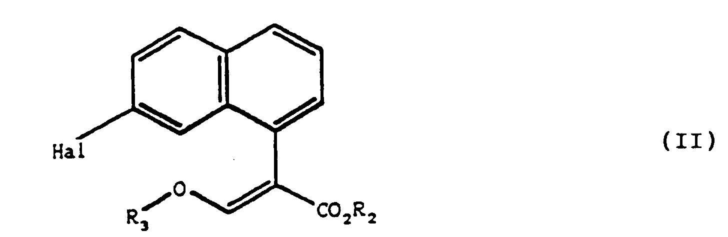 in which Hal represents a halogen atom and R₂ and R₃ retain the same meaning as previously, is subjected to the action of a compound of formula (III):
RC≡CH (III)
in which R retains its previous meaning, in order to obtain the compound of formula (I), which if desired and if R comprises a hydroxyl function, is subjected to a functionalization agent in order to obtain the corresponding compound of formula (I).

21. Process according to claim 20, characterized in that in addition a compound of formula (I) in which R represents a hydrogen atom is subjected to the action of a compound of formula ArHal, in which Ar represents an optionally substituted aryl or heteroaryle radical and Hal represents a halogen atom in order to obtain the corresponding compound of formula (I) in which R represents an optionally substituted aryl or heteroaryl radical.

22. As new chemical products, the compounds of formula (II) as defined in claim 20.

23. As a new chemical product as defined in claim 22, methyl 7-bromo alpha-(methoxymethylene) 1 -naphthalene acetate.
